# EUROPEAN PATENT SPECIFICATION

(11) **EP 1 812 394 B1**
(45) Date of publication and mention of the grant of the patent: **02.03.2011**
(21) Application number: 04770672.6
(22) Date of filing: 19.07.2004
(51) Int. Cl.: C07D 215/18

(54) **PROCESS FOR THE PREPARATION OF MONTELUKAST AND ITS SALTS**
VERFAHREN ZUR HERSTELLUNG VON MONTELUKAST UND SALZEN DAVON
PROCEDE DE PREPARATION DE MONTELUKAST ET DE SES SELS

(43) Date of publication of application: 01.08.2007
(73) Proprietor: Matrix Laboratories Ltd, Secunderabad 500 003, Andhra Pradesh (IN)
(72) Inventor: CHAVA, Satyanarayana, Secunderabad 500 009 (IN); GORANTLA, Seeta, Ramanjaneyulu, Secunderabad 500 015 (IN); INDUKURI, Venkata, Sunil, Kumar, Kukatpally, Hyderabad 500 072 (IN); SIMHADRI, Srinivas, Kukatpally, Hyderabad 500 072 (IN); JAMMULA, Vera, Venkata, Krishna, Kishore, Kukatpally, Hyderabad 500 072 (IN)
(74) Representative: Wittkopp, Alexander
(86) International application number: PCT/IN2004/000212
(87) International publication number: WO 2006/008751

(56) References cited:
- EP-A1- 0 480 717
- WO-A1-95/18107
- WO-A1-96/22987
- WO-A1-03/066598
- WO-A1-2005/040123
- US-A1- 2005 107 612

## Description

The present invention relates to a process for the preparation of 1-[[[(1R)-1-[3-[(1E)-2-(7-chloro-2-quinolinyl) ethenyl] phenyl] -3- [2-(1-hydroxy-1-methylethyl) phenyl] propyl] thio] methyl] cyclopropaneacetic acid, its alkali salts (Montelukast alkaline salts), using novel compounds namely Methyl 2-[(3S)-[3-[(2E)-(7-chloro quinolin-2-yl) ethenyl] phenyl]-3-halopropyl] benzoate, 1-[[[(1R)-1-[3-[(1E) -2-(7-chloro-2-quinolinyl) ethenyl] phenyl] -3-[2-(1-hydroxy-1-methylethyl) phenyl] propyl] thio] methyl] cyclopropaneacetic acid organic base salts (Montelukast organic base salts).

Montelukast sodium namely Sodium salt of 1-[[[(1R)-1-[3-[(1E)-2-(7-chloro-2-quinolinyl) ethenyl] phenyl]-3-[2-(1-hydroxy-1-methylethyl) phenyl] propyl] thio] methyl] cyclopropaneacetic acid has the formula

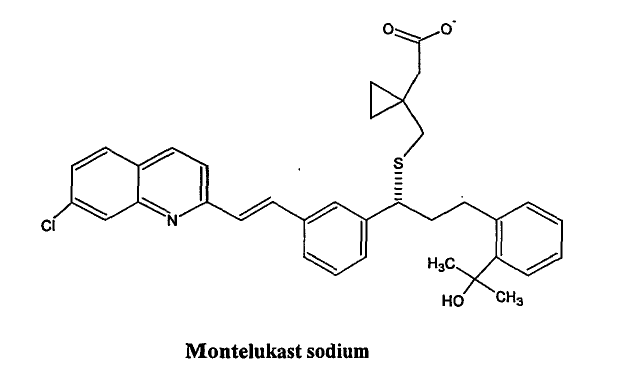

Montelukast sodium is a leukotriene antagonist and inhibits the synthesis of leukotriene biosynthesis. It is useful as anti-asthmatic, anti-allergic, anti-inflammatory, cytoprotective agent and hence useful in the treatment of angina, cerebral spasm, glomerular nephritis, hepatic, end toxemia, uveitis and allograft rejection.

European Patent No 480,717 discloses Montelukast sodium along with other related compounds and the methods for their preparation. The reported method of synthesis proceeds through corresponding methyl ester namely, Methyl 2-[(3S)-[3-[(2E)-(7-chloroquinolin-2-yl) ethenyl] phenyl]-3-hydroxypropyl] benzoate and involves coupling methyl 1-(mercaptomethyl) cyclopropaneacetate with a mesylate generated in-situ. The methyl ester of Montelukast is hydrolyzed to free acids and the latter converted directly to Montelukast sodium salt (Scheme-1). The process is not particularly suitable for large-scale production because it requires tedious chromatographic purification of the methyl ester intermediate and / or the final product and the product yield is low.

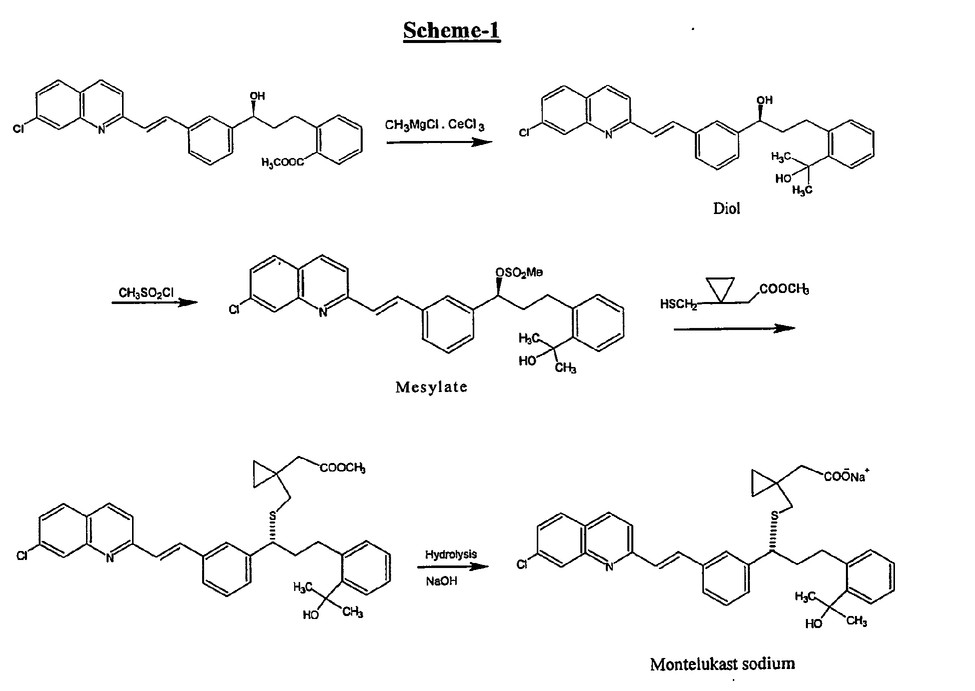

US Patent No 5,614,632 discloses that the products obtained as per EP 480,717 are amorphous sodium salts, which are hydrated and often not ideal for pharmaceutical formulation and therefore provides an improved process for the preparation of crystalline Montelukast sodium which comprises of the following steps (Scheme - 2):
■ Reaction of methyl 2-[3(S)-[3-[2-(7-chloroquinolin-2-yl) ethenyl] phenyl]-3-hydroxypropyl benzoate (I) with Grignard reagent, methyl magnesium chloride in presence of cerium chloride to give Diol (II)
■ Reaction of Diol (II) with methane sulfonyl chloride to afford 2-[2-[3(s)-[3-(2-(7-chloro quinolin-2-yl) ethenyl] phenyl]-3-methane sulfonyloxy propyl] phenyl]-2-propanol (III)
■ Condensation of 2-[2-[3(s)-[3-(2-(7-chloro quinolin-2-yl) ethenyl] phenyl]-3-methane sulfonyloxypropyl] phenyl]-2-propanol (III) with dilithium anion of 1-mercaptomethyl) cyclopropaneacetic acid, which has been generated by the reaction of 1-(mercaptomethyl) cyclopropaneacetic acid (IV) with n-Butyl lithium
■ Isolation of the condensed product, Montelukast as solid Montelukast dicyclohexylamine salt
■ Purification and conversion of Montelukast dicyclohexylamine salt into Montelukast sodium
■ Crystallization of Montelukast sodium from a mixture of toluene and acetonitrile US Patent No 5,614,632 further discloses the two polymorphic forms (Form'A' and Form'B') of Montelukast dicyclohexyl amine salt and a process for the preparation by recrystallization from mixture of solvents, which were characterized by FTIR, XRD pattern.

The reaction of Diol (II) with methane sulfonyl chloride involves the reaction temperature of about -25°C and the storage condition of the intermediate, 2-[2-[3(s)-[3-(2-(7-chloro quinolin-2-yl) ethenyl] phenyl]-3-methane sulfonyloxypropyl] phenyl]-2-propanol (III) at temperature below -15°C for having the stability. The process further involves the reaction, formation of dilithium anion of 1-(mercaptomethyl) cyclopropaneacetic acid which requires the usage of n-Butyl lithium, a highly flammable and hazardous reagent and the reaction is at temperature below -5°C further requires anhydrous conditions.

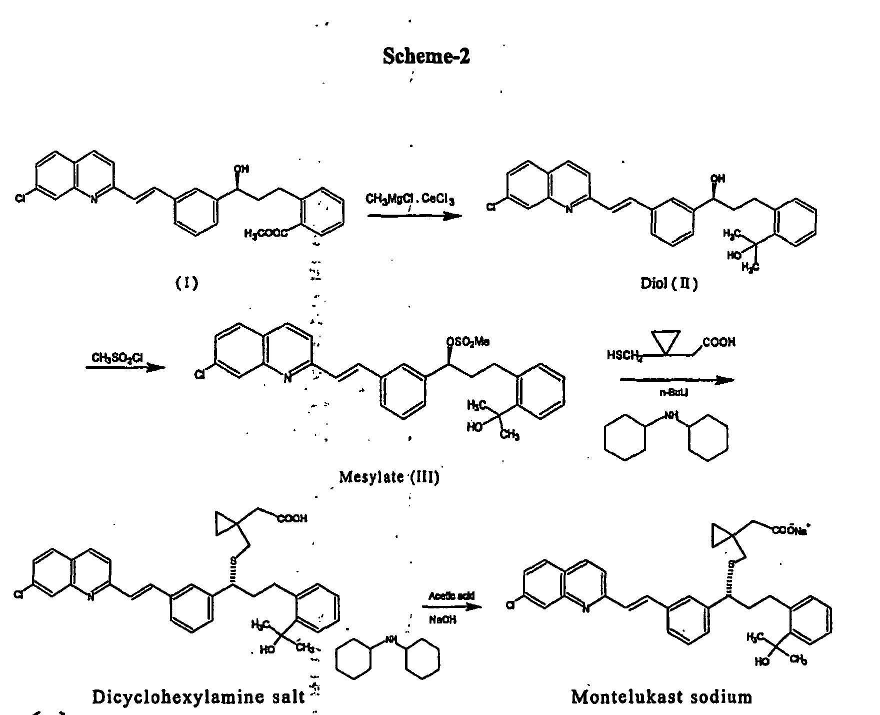

US 2005/0107612 describes the utilization of 2-[1-[1-R-3-[2-(7 chloro quinolin-2-yl) vinyl [phenyl]-3-[2-methoxy carbonyl phenyl]propyl sulfonyl methyl]cyclo propyl]acetic acid in the synthesis of Montelukast's free acid, starting earlier from 2-(3-R-(3-(2-(7-chloro 2-quinolinyl)-ethenyl)-3 hydroxy propyl) benzoate, wherein the methoxycarbonylphenyl group is treated with methylmagnesiumchloride to give the propane-2-ol group.

It is a long felt of the industry to provide a process which avoids the usage of low temperature reactions viz. below, -25°C, storage conditions of viz below -15°C, unstable intermediate (III), handling of highly flammable, hazardous reagents for the preparation of Montelukast alkali salts and involves the isolation of a stable crystalline solid Montelukast free acid which can be purified by various methods.

The main object of the present invention is to provide a new process for the preparation of stable crystalline Montelukast, its organic base salts without involving the unstable or limited stable intermediates and further conversion to Montelukast alkali salts.

Another object of the invention is to provide a process for the preparation of Montelukast and its alkali salts without involving the low temperature (-25°C) reactions and storage conditions at lower temperature (-15°C).

Another object of the present invention is to provide a process for the preparation of Montelukast and its alkali salts using Methyl 2-[(3S)-[3-[(2E)-(7-chloroquinolin-2-yl) ethenyl] phenyl]-3-halopropyl] benzoate.

Another object of the present invention is to provide a process for the preparation of Montelukast and its alkali salts using 2-[2-[3S-[3-[ 2-(7-chloroquinolin-2-yl) ethenyl] phenyl]-3-halopropyl] phenyl]-2-propanol.

Another object of the present invention is to provide a process for the preparation of Montelukast and its alkali salts using 2-[1-[1(R)-[3-[2-(7-chloroquino in-2-yl) ethenyl] phenyl]-3-[2-(methoxycarbonyl) phenyl] propylsulfanylmethyl]cyclopropane] acetic acid.

Another object of the present invention is to provide a process for the preparation of novel Montelukast organic base salts.

Another object of the present invention is to provide a process for the purification of crystalline Montelukast free acid via novel organic base salts.

Yet another object of the invention is to provide novel compounds and their use in the preparation of Montelukast and its alkali salts.

Yet another object of the present invention is to provide novel compounds 2-[2-[3S-[3-[ 2-(7-chloroquinolin-2-yl)ethenyl]phenyl]-3-halopropyl] phenyl]-2-propanol and their use for preparation of Montelukast and its alkali salts.

Another object of the invention is to provide fingerprinting of the novel intermediate 2-[1-[1(R)-[3-[2-(7-chloroquinolin-2-yl) ethenyl] phenyl]-3-[2-(methoxy carbonyl) phenyl] propyl sulfanylmethyl] cyclopropane] acetic acid using NMR, mass and IR spectral data.

Another object of the invention is to provide fingerprinting of the novel intermediates 2-[2-[3S-[3-[2-(7-chloroquinolin-2-yl) ethenyl] phenyl]-3-halopropyl] phenyl] -2-propanol using NMR, mass and IR spectral data.

Yet another object of the invention is to provide a finger printing of crystalline Montelukast free acid using IR, X-ray diffraction pattern.

Yet another object of the present invention is to provide novel Montelukast organic amine salts for their use in preparation of Montelukast alkali salts.

Yet another object of the present invention is to provide the finger printing of the novel Montelukast organic amine salts by NMR, IR and X-ray diffraction pattern

Accordingly, the present invention relates to method for the preparation of Montelukast and its alkali salts from Methyl 2-[(3S)-[3-[(2E)-(7-chloro quinolin-2-yl) ethenyl] phenyl]-3-halopropyl] benzoate (Halo ester, V) by two alternate routes (Scheme - 3 & Scheme - 4).

As illustrated in Scheme-3, Methyl 2-[(3S)-[3-[(2E)-(7-chloroquinolin-2-yl) ethenyl] phenyl]-3-halopropyl] benzoate (V) on condensation with 1-(mercatpomethyl) cyclopropane acetic acid (IV) in presence of alkali hydride or alkoxide affords 2-[1-[1(R)-[3-[2-(7-chloroquinolin -2-yl) ethenyl] phenyl]-3-[2-(methoxy carbonyl) phenyl] propyl sulfanyl methyl] cyclopropane]acetic acid (VI) which on reaction with Grignard reagent methyl magnesium chloride in presence of cerium chloride or methyl magnesium bromide affords the Montelukast which can isolated as free acid or optionally as Montelukast amine salt.

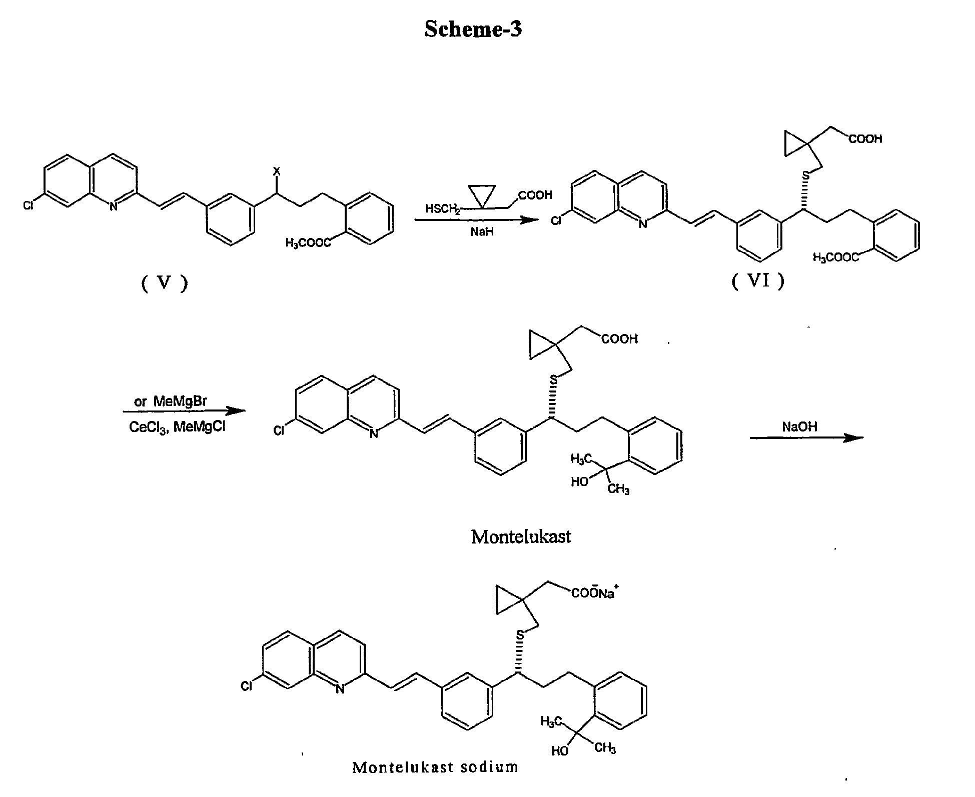

In the other route as illustrated in Scheme - 4, Methyl 2-[(3S)-[3-[(2E)-(7-chloro quinolin-2-yl) ethenyl] phenyl]-3-halopropyl] benzoate (V) on reaction with Grignard reagent, methyl magnesium chloride in presence of cerium chloride or methyl magnesium bromide affords the novel intermediate 2-[2-[3S-[3-[2-(7-chloroquinolin-2-yl) ethenyl] phenyl]-3-halopropyl] phenyl]-2-propanol (VII) which on condensation with 1-(mercapto methyl)cyclopropane acetic acid (IV) in presence of alkali hydride or alkoxide followed by neutralization affords the Montelukast which can be isolated as free acid or as Montelukast organic base salt (Scheme-4).

The Montelukast free acid, Montelukast amine salts can be purified, converted into Montelukast free acid or the required Montelukast alkali salts by following the similar procedure reported in the literature.

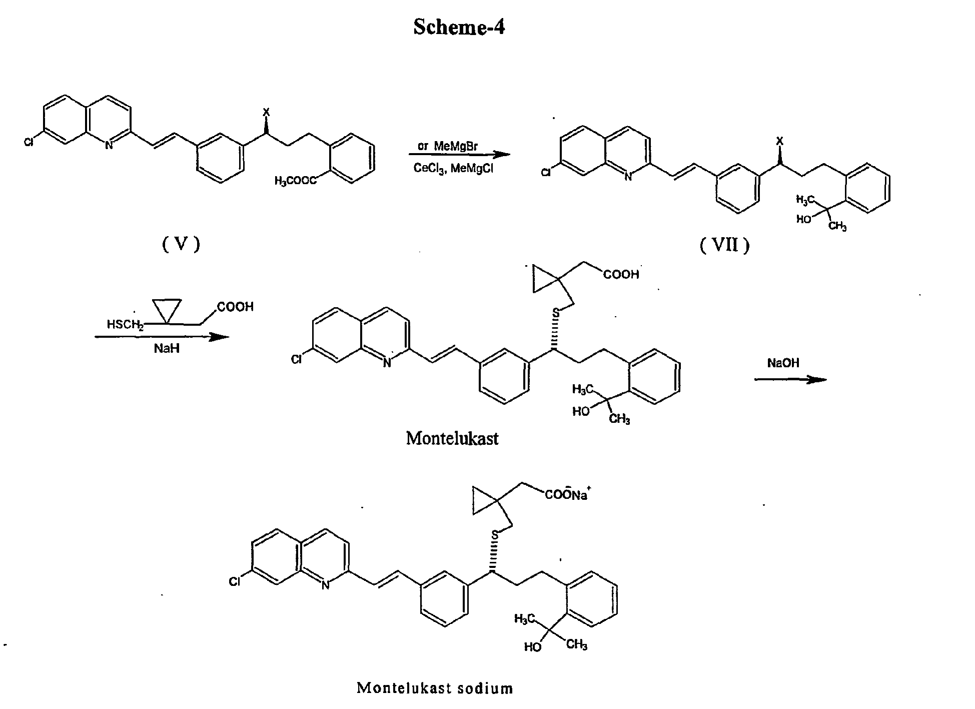

### Brief description of the drawings:

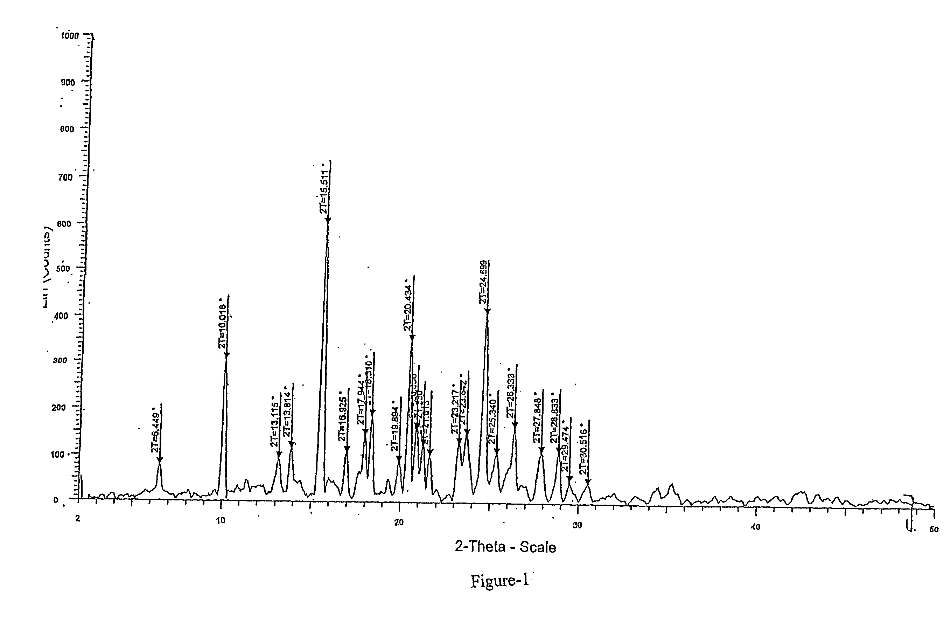

Fig.1 X-Ray diffraction pattern of the Montelukast free acid

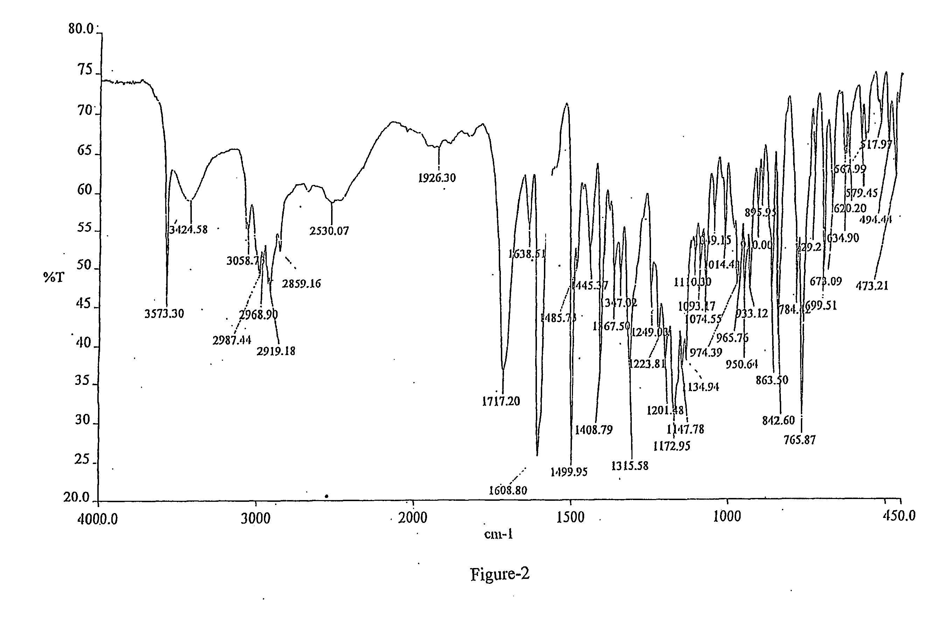

Fig.2 FTIR spectrum of the Montelukast free acid

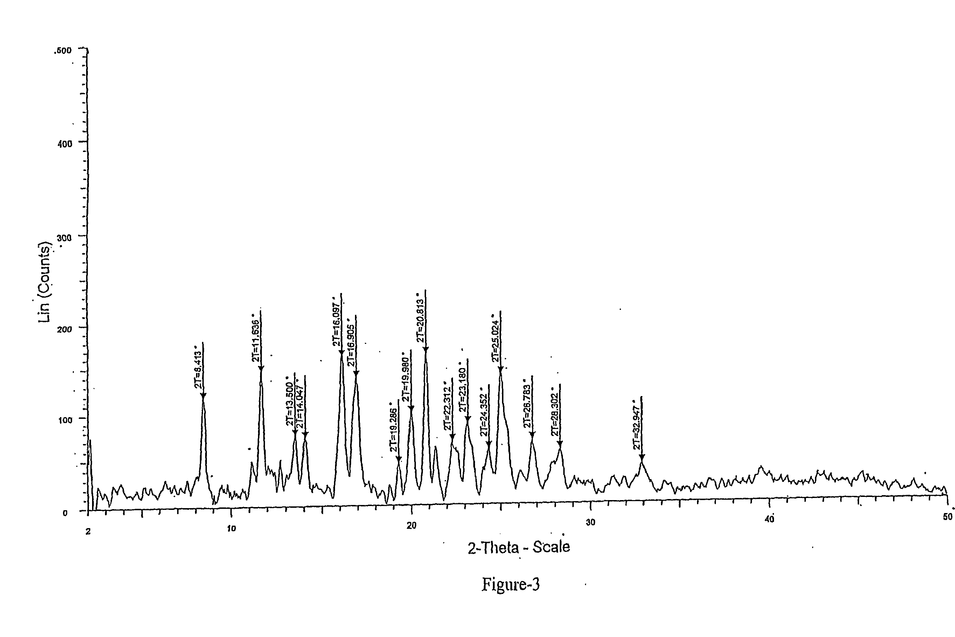

Fig.3 X-ray diffraction pattern of the Montelukast dipropylamine salt

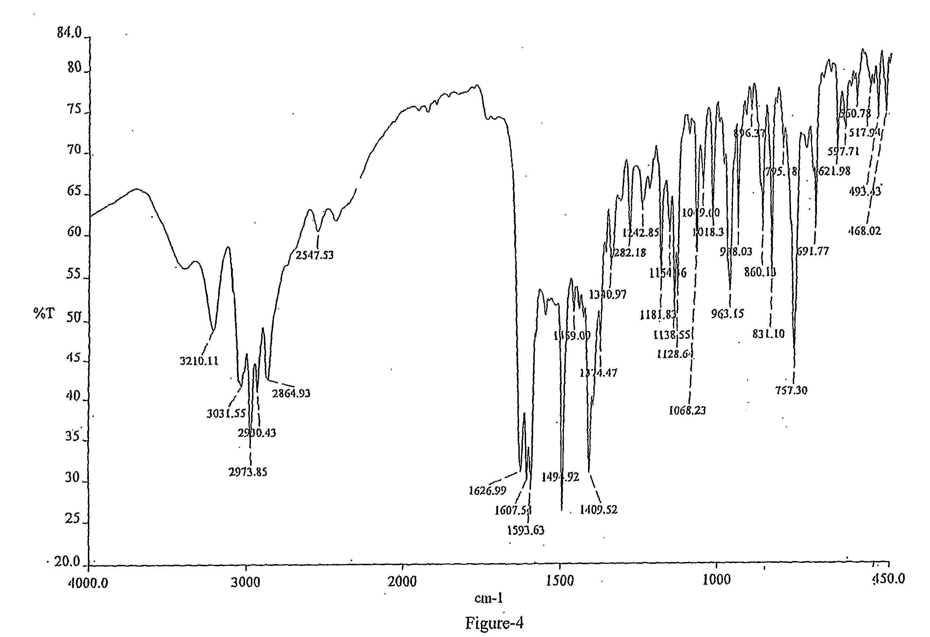

Fig.4 FTIR spectrum of the montelukat dipropylamine salt

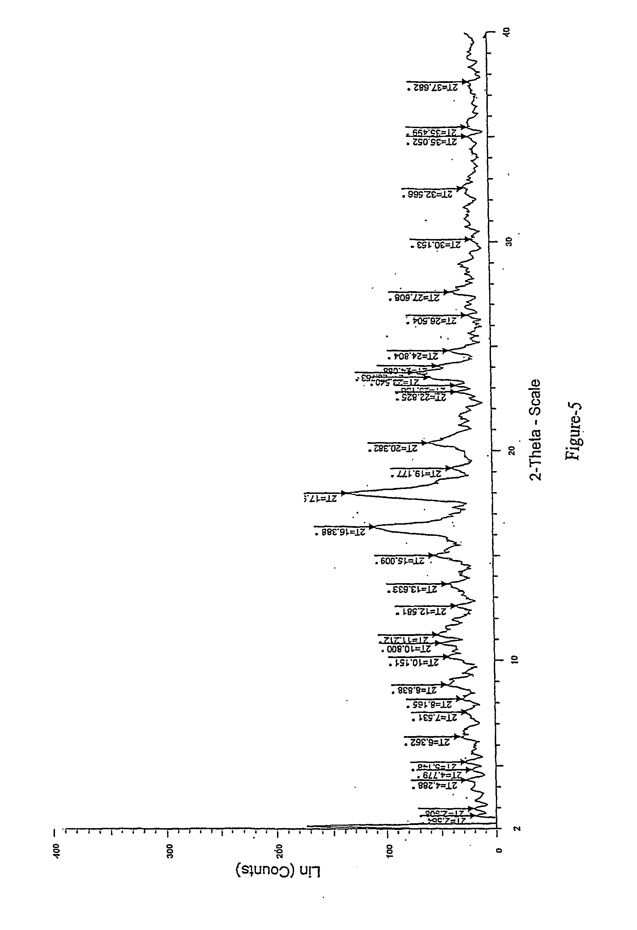

Fig.5 X-ray diffraction pattern of the Montelukast alpha -methyl benzyl amine salt

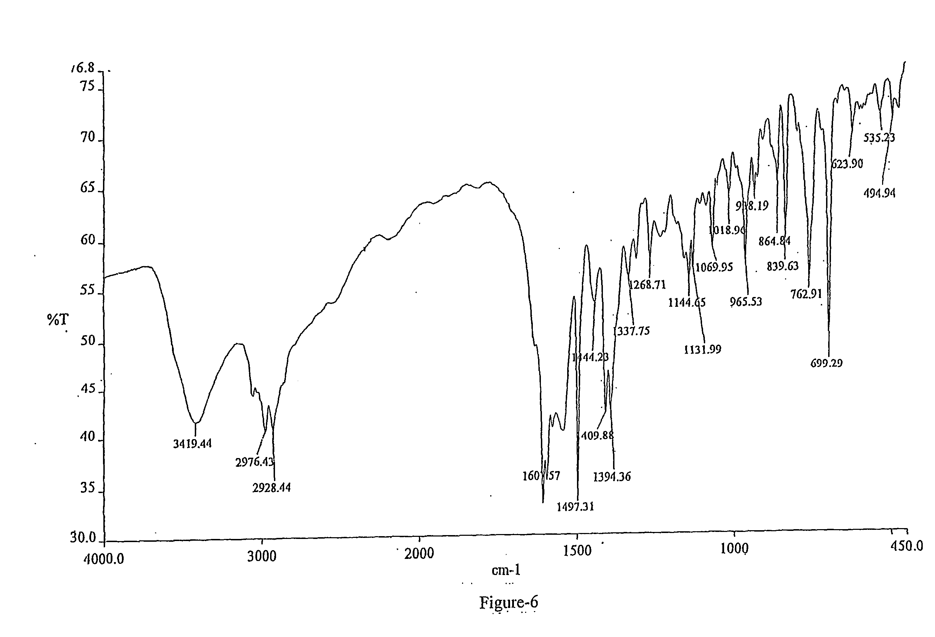

Fig.6 FTIR spectrum of the Montelukast alpha -methylbenzylamine salt

The process of the present invention for the preparation of Montelukast and its salts comprises of:
■ Reacting Methyl 2-[(3S)-[3-[(2E)-(7-chloro quinolin-2-yl) ethenyl] phenyl]-3-halopropyl] benzoate (V) with 1-(mercapto methyl) cyclopropane acetic acid (IV) in presence of alkali hydride or alkoxide
■ Isolating the product 2-[1-[1(R)-[3-[2-(7-chloroquinolin-2-yl) ethenyl] phenyl]-3-[2-(methoxycarbonyl) phenyl] propylsulfanylmethyl] cyclopropane] acetic acid (VI), a novel intermediate as dicyclohexylamine salt
■ Neutralizing the 2-[1-[1(R)-[3-[2-(7-chloroquinolin-2-yl) ethenyl] phenyl] -3-[2-(methoxycarbonyl) phenyl] propyl sulfanylmethyl] cyclopropane acetic acid dicyclohexyl amine salt followed by reaction with Grignard reagent to give Montelukast free acid
■ Isolating the Montelukast as crystalline free acid or optionally as Montelukast organic base salt by reaction with organic amines

Optionally the Process comprises:
■ Reacting Methyl 2-[(3S)-[3-[(2E)-(7-chloro quinolin-2-yl) ethenyl] phenyl]-3-halopropyl] benzoate (V) with Grignard reagent to afford 2- [2- [(3S)- [3- [(2E)- (7-chloroquinolin-2-yl) ethenyl] phenyl]-3-halopropyl] phenyl-2-propanol (VII)
■ Condensing 2-[2-[(3S)-[3-[(2E)-(7-chloro quinolin-2-yl) ethenyl] phenyl]-3-chloropropyl] phenyl-2-propanol (VII) with 1-(mercapto methyl) cyclopropane acetic acid (IV) in presence of alkali hydride or alkoxide
■ Isolating the formed product, Montelukast as free acid or as Montelukast organic base salts
■ Converting Montelukast organic base salts into Montelukast free acid and or its required alkali salt

The prepared 2-[1-[1(R)-[3-[2-(7-chloroquinolin-2-yl] ethenyl] phenyl] -3-[2-(methoxycarbonyl) phenyl] propylsulfanylmethyl] cyclopropane] acetic acid (VI), 2-[2-[(3S)-[3-[(2E)-(7-chloroquinolin-2-yl) ethenyl] phenyl]-3-halo propyl] phenyl-2-propanol (VII) are novel intermediates, characterized by chemical analysis, NMR, Mass and IR spectral data.

The novel crystalline anhydrous Montelukast is characterized by chemical analysis, NMR, IR spectral and XRD.

The starting material 1-(mercaptomethyl) cyclopropaneacetic acid is prepared by the literature reported method.

Condensation of Methyl 2-[(3S)-[3-[(2E)-(7-chloroquinolin-2-yl) ethenyl] phenyl]-3-halopropyl] benzoate solution in DMF with a mixture of 1-(mercapto methyl) cyclopropane acetic acid, alkali hydride or alkali alkoxide (where the preferable alkali hydride is sodium hydride, alkali alkoxide is the potassium tertiary butoxide) in DMF is carried out at temperature of about -15°C to 10°C preferably -5°C to 5°C for about 12 to 30 hrs followed by quenching of the reaction mass into a mixture of water, ethyl acetate, adjusting the pH of the reaction mass to neutral with tartaric acid, separating the layers, extracting of aqueous layer with ethyl acetate, washing the combined organic layer with tartaric acid, water, drying over dehydrating agents, concentrating the organic layer under reduced pressure followed by slow addition of dicyclohexyl amine at temperature 30° C to 10° C followed by mixing for about 16 hrs to 48 hrs, adding n-hexane, isolating and drying yields 2-[1-[1(R)-[3-[2-(7-chloroquinolin-2-yl) ethenyl]phenyl]-3-[2-(methoxycarbonyl) phenyl]propylsulfanyl methyl] cyclopropaneacetic acid dicyclohexylamine salt.

2-[1-[1(R)-[3-[2-(7-chloroquinolin-2-yl) ethenyl] phenyl]-3-[2- (methoxycarbonyl) phenyl] propylsulfanylmethyl] cyclopropane acetic acid dicyclohexylamine salt on acidification with acetic acid in mixture of water, methylene chloride, separation of layers followed by washing with water, drying over dehydrating agents and removal solvent gives the 2-[1-[1(R)-[3-[2-(7-chloroquinolin-2-yl) ethenyl] phenyl]-3-[2-(methoxycarbonyl) phenyl] propyl sulfanylmethyl] cyclopropaneacetic acid (VI).

Adding slowly methyl magnesium chloride to a suspension of cerium chloride in THF at temperature of -5°C to 5°C followed by maintenaning at -5°C to 5°C for about 2 hrs, further addition of solution of 2-[1-[1(R)-[3-[2-(7-chloroquinolin-2-yl) ethenyl] phenyl]-3-[2-(methoxycarbonyl) phenyl] propyl sulfanylmethyl] cyclopropane acetic acid (VI) in toluene at temperature of -5°C to 5°C, maintaining for about 2 to 8 hrs, quenching the reaction mass into a mixture of dilute acetic acid: ethyl acetate at temperature below 20°C, separating the layers, washing the organic layer with sodium carbonate solution, sodium chloride solution, drying over dehydrating agents, removing the ethyl acetate by distillation to get a residue, dissolving the residue obtained in ethanol, gradual cooling to ambient temperature followed by seeding, mixing for about 20 hrs to 28 hrs, isolating and drying affords the Montelukast free acid.

As depicted in Scheme-4, adding the methyl magnesium chloride to the suspension of cerium chloride in THF followed by maintenaning at -5°C to 0°C for about 2 to 3 hrs, adding the dehydrated solution of Methyl 2-[(3S)-[3-[(2E)-(7-chloroquinolin-2-yl) ethenyl] phenyl]-3-halopropyl] benzoate (V) in toluene followed by maintaining and quenching with dilute acetic acid solution, extracting, washing the organic layer with sodium carbonate solution, sodium chloride solution, crystallizing from ethyl acetate yields the 2-[2-[(3S)-[3-[(2E)-(7-chloroquinolin-2-yl)ethenyl] phenyl]-3-halopropyl]phenyl-2-propanol (VII).

Addition of 1-(mercaptomethyl) cyclopropaneacetic acid (IV) solution in DMF to a suspension of alkali hydride or alkali alkoxide (the preferable alkali hydrides are sodium hydride, alkoxides are sodium methoxide, potassium tertiary butoxide) in DMF/ THF at temperature of -10°C to 10°C, preferably about -5°C to 0°C, mixing for about 30 min. to 3 hrs, slow addition of solution of 2-[2-[(3S)-[3-[(2E)-(7-chloroquinolin-2-yl) ethenyl] phenyl]-3-halopropyl] phenyl-2-propanol (VII) in DMF over 30 min. to 4 hrs at temperature of -10°C to 10°C preferably about -5°C to 0°C, mixing for about 6 hrs to 24 hrs preferably for 10 hrs to 16 hrs, transferring the reaction mass to a mixture of water : ethyl acetate, adjusting the pH of the reaction mass to neutral with tartaric acid, separating the layers, extracting the aqueous layer with ethyl acetate, washing the combined organic layer with tartaric acid solution followed by successive water washings, drying over dehydrating agents, concentrating the solution under reduced pressure followed by adding ethanol followed by mixing at reflux temperature for about 10 min. to 2 hrs followed by cooling, seeding and maintaining at 15°C to 40°C for about 30 min to 24 hrs yields the Montelukast free acid.

Optionally the Montelukast may be isolated as Montelukast organic base salts by dissolving the residue in ethyl acetate after distilling the ethyl acetate, treating with organic amine such as diisopropylamine, dipropylamine, tributylamine, dibenzylamine, dicyclohexylamine, alpha-methylbenzylamine, selectively dicyclohexylamine, dipropylamine, at temperature 10°C to 30°C followed by maintenaning for about 16 hrs to 48 hrs, adding hexane and mixing for another 16 hrs to 30 hrs yields the Montelukast organic base salt.

Montelukast free acid, Montelukast organic base salts can be purified, converted into required Montelukast alkali salts as follows.

Suspending the Montelukast organic salts in a mixture of water: methylene chloride, adding dilute acetic acid, separating the layers, washing the organic layer with water, drying over dehydrating agents, adding the sodium hydroxide solution in ethanol to the dried organic layer, followed by removing the solvents under reduced pressure at temperature below 40°C to gives the residue which on dissolving in toluene followed by transferring the solution into n-Heptane, isolating and drying affords Montelukast sodium.

Suspending the Montelukast organic salts in a mixture of water: methylene chloride, adding dilute acetic acid, separating the layers, washing the organic layer with water, drying over dehydrating agents, removing the methylene chloride at temperature below 40°C, dissolving the residue in ethyl acetate by raising the temperature 40°C to reflux temperature followed by gradual cooling 20°C to 25°C, isolating and drying at temperature of 30°C to 50°C preferably at 40°C to 50°C gives the Montelukast free acid.

Montelukast organic base salts can be prepared from Montelukast free acid by dissolving the Montelukast free acid in ethyl acetate, adding the organic base selectively dicyclohexyl amine, dipropyl amine, diisopropylamine, dibenzylamine, alpha-methylbenzylamine at temperature of 20°C - 35°C followed by mixing for about 10hrs to 36 hrs, adding the second solvent selectively hydrocarbon of C-5 to C-7, acetonitrile, ethers of C-4 to C-8, mixing for about 2 hrs to 18 hrs, isolating and drying. The preferred hydrocarbon is n-hexane, n-Heptane, toluene, cyclohexane, and methyl cyclohexane. The preferred ether is diethyl ether, di-isopropyl ether.

Montelukast sodium can be prepared from Montelukast free acid by dissolving the Montelukast free acid in methanol by raising temperature to 40°C to 55°C, cooling to 20°C to 35°C, adding the sodium hydroxide solution in ethanol, mixing for about 30 min., followed by removing the solvents under reduced pressure at temperature below 40°C to get a residue. The residue on dissolving in toluene followed by pouring into n-Heptane gives the Montelukast sodium.

The invention is now illustrated with a few non-limiting examples.

### Example-1: Preparation of 2-[1-[1(R)-[3-[2-(7-Chloroquinolin-2-yl) ethenyl] Phenyl]-3- [2-(methoxycarbonyl) phenyl] propylsulfanylmethyl] cyclopropylacetic acid dicyclohexylamine salt (DCHA salt)

Sodium hydride (28 g, 0.70 moles) is suspended in DMF (400 ml), cooled to -5°C under nitrogen and solution of 1-(mercaptomethyl) cyclopropaneacetic acid (46 g, 0.315 mole) in DMF (100 ml) is slowly added the at -5°C to 0°C over 1 hr and maintained at -5°C to 0°C for 1 hr. Methyl 2-[(3S)-[3-[(2E)-(7-chloro quinolin-2-yl) ethenyl] phenyl]-3-chloropropyl] benzoate (100 g, 0.21 mole) in 4 equal lots is then slowly added at -5°C to 0°C over 1hr and the reaction mass is maintained at -5°C to 0°C for 24 hrs. The reaction mass is transferred into a mixture of water (1000 ml): ethyl acetate (1000 ml) and mixed for 30 min. at temperature below 20°C. pH of the reaction mass is adjusted to 7.0 by addition of 20% aqueous solution of Tartaric acid (100 ml) at 10°C-25°C and mixed for 30 min. The layers are allowed to settle, the organic layer separated. The aqueous layer is extracted with ethyl acetate (1000 ml). The organic layer and ethyl acetate extract are combined, washed with 5% aqueous tartaric acid solution (400 ml) and water (2 x 200 ml), dried over sodium sulphate, treated with activated carbon for 30 min at 25°C - 35°C and ethyl acetate is distilled off under reduced pressure at temperature below 40°C to get the residue which is dissolved in ethyl acetate (600 ml) by heating to 45°C, cooled to 20°C and slowly added the dicyclohexylamine (42 ml, 0.21 mole) over 30 min. at 20°C. The temperature is maintained at 20°C - 22°C for 1 hr, seeded with 2-[1-[1(R)-[3-[2-(7-Chloroquinolin-2-yl) ethenyl] Phenyl]-3-[2-(methoxycarbonyl) phenyl] propyl sulfanylmethyl] cyclopropyl] acetic acid dicyclohexylamine salt (200 mg) and maintained at 20°C - 25°C for 36 hrs. n-Hexane (1200 ml) is added over 40 min and the reaction mass mixed for 24 hrs at 20°C - 25°C. The solid is filtered, washed with n-Hexane (500 ml) and dried at 40°C - 45°C to constant weight.

Dry wt of the product is 85 g, (yield of 52.8%).
Elemental analysis: C: 71.27%, H: 7.72%, N: 3.83%, S: 4.58% and calculated values for C₄₆H₅₅ClN₂O₄S C: 71.99%, H: 7.22%, N: 3.65, S: 4.18%
IR Spectrum (KBr, em-¹): 3423, 2934, 2855, 1715, 1626, 1607, 1534, 1497, 1449, 1410, 1387, 1342, 1311, 1255, 1189, 1129, 1081, 1067, 1015, 964, 929, 837, 755, 695
¹HNMR (300 MHz, CDCl₃, ppm): 8.06 - 8.12 (m, 2H), 7,83 -7.86 (m, 1H), 7.66 - 7.72 (m, 3H), 7.20 - 7.49(m 9H), 3.90 - 3.95 (m, 1H), 3.81 (s, 3H), 2.89 - 3.02 (m, 2H), 2.74 - 2.83 (m, 2H), 2.57(s, 2H), 2.37-2.38(m, 2H), 2.12 - 2.21 (m, 2H), 1.94 - 1.97 (m, 4H), 1.74 - 1.77 (m, 4H), 1.60 -1.63 (d, 2H), 1.12 -1.35 (m, 10H), 0.35 - 0.57 (m, 4H).
Mass Spectrum (M+): 587.2

### Example-2: Preparation of 1-[[[(1R)-1-[3-[(1E)-2-(7-chloro-2-quinolinyl) ethenyl] phenyl]-3-[2-(1-hydroxy-1-methylethyl) phenyl] propyl] thio] methyl] cyclopropaneacetic acid (Montelukast free acid)

### Step-1:

2-[1-[1(R)-[3-[2-(7-Chloroquinolin-2-yl) ethenyl] phenyl]-3-[2-(methoxycarbonyl) phenyl] propylsulfanylmethyl] cyclopropylacetic acid dicyclohexylamine salt (140 g, 0.21 mole) is suspended in a mixture of methylene chloride (1680 ml), water (980 ml) and mix for 15min. Adjusted the pH of the reaction mass to 4.5 with of 6% acetic acid (240 ml) at 25°C - 35°C, and mixed for 30 min, allowed to settle the layers, separated the organic layer and extracted the aqueous layer with methylene chloride (1000 ml). Combined the organic layers, washed with water (980 ml), dried over sodium sulphate and distilled off methylene chloride finally under reduced pressure to get the residue. Dissolved the residue in toluene (1000 ml) and used the solution to next step.

### Step-2:

Cerium chloride (50g) is suspended in THF (1050 ml), raised the temperature of the suspension and distilled off initially 50 ml of THF and maintained the mass at reflux temperature (65°C) for 3 hrs under nitrogen atmosphere. Cooled the reaction mass to -5°C, added 3.0 molar methyl magnesium chloride solution in THF (500 ml) at temperature -5°C - 0°C over 40 min and maintained for 2 hrs at that temperature. Slowly added the step-1 solution over 60 min and maintained at 0°C - 5°C for 6 hrs. Transferred the reaction mass into a pre cooled mixture of 12% acetic acid (1400 ml): ethyl acetate (800 ml) at temperature below 20°C and mixed for 30 min at 18°C - 20°C. Allowed the mass for settling, separated the organic layer, extracted the aqueous layer with ethyl acetate (800 ml), combined the organic layers, washed successively with 10% sodium carbonate solution (1600 ml), 5 % sodium chloride solution (2 x 1000 ml) and dried the organic layer over anhydrous sodium sulphate (15g). Treated the dried organic layer with activated carbon; distilled off ethyl acetate from the clear solution at temperature below 45°C under reduced pressure to get the residue. Added ethanol (200 ml) to the residue; raised the temperature to reflux for 30 min. to get a clear solution. Gradually cooled the reaction mass to 28°C - 32°C, seeded with Montelukast free acid (500 mg) and maintained at 28°C - 32°C for 24 hrs. Cooled the reaction mass to 20°C and maintained at 20°C for 1 hr. Filtered the product, washed with chilled ethanol (50 ml) and dried at 45°C - 50°C till constant weight.

The dry weight of the Montelukast free acid is 40 g (yield is 52.3 %)
Elemental analysis: C: 70.50%, H: 6.25%, N: 2.44%, S: 5.38% and calculated values for C₃₅H₃₆ClNO₃S C: 71.7%, H: 6.19%, N: 2.39%, S: 5.47%
IR Spectrum (KBr, cm-¹): 3417, 2973, 2925, 1707, 1608, 1498, 1441, 1313, 1223, 1145, 1074, 963, 937, 863, 838, 762, 697 ¹H NMR (300 MHz, CDCl₃, ppm): 8.11 (d, 1H), 8.07(d, 1H), 7.73 (brs, 1H), 7.61 - 7.74 (m, 4H), 7.45 (m, 1H), 7.43 - 7.53 (dd, 1H), 7.33 - 7.43 (m, 3H), 7.10 - 7.20 (m, 3H), 5.02 - 5.07 (t, 1H), 3.0 - 3.35 (m, 2H), 2.65 (m, 2H), 2.40 - 2.54 (m, 2H), 2.39 (m, 2H), 1.63 (2s, 6H), 0.51 - 0.61 (m, 4H).
Mass Spectrum (M+): 586.2

### Example-3: Preparation of 1-[[[(1R)-1-[3-[(1E)-2-(7-chloro-2-quinolinyl) ethenyl] phenyl]-3-[2-(1-hydroxy-1-methylethyl) phenyl] propyl] thio] methyl] cyclopropaneacetic acid dicyclohexylamine salt (Montelukast DCHA salt)

Montelukast dicyclohexyl amine salt is prepared by reaction of 2-[1-[1(R)-[3-[2-(7-Chloroquinolin-2-yl) ethenyl] phenyl]-3-[2-(methoxycarbonyl) phenyl] propylsulfanyl methyl] cyclopropyl] acetic acid with methyl magnesium chloride in presence of cerium chloride by following similar procedure as in step-I, step-2 of the Example-II and carried out isolation procedure as follows.

After distillation of ethyl acetate from the combined dried organic layer, added ethyl acetate (200 ml) to the residue and again distilled off under reduced pressure to get the solid. To the residual solid added ethyl acetate (600 ml) at temperature 40°C - 45°C, cooled to 20°C, slowly added the dicyclohexylamine (42 ml, 0.21 mole) over 30 min at 20°C - 25°C, maintained for 1 hr at temperature 20°C - 25°C, seeded with Montelukast DCHA salt and maintained at 20°C - 25°C for 24 hrs. Slowly added n-hexane (1200 ml) over 1hr and maintained the reaction mass at 20°C - 25°C for 24 hrs. Filtered the product, washed with n-hexane (500 ml) and dried at 45°C - 50°C till constant weight.

The dry weight of the Montelukast DCHA salt is 50 g (yield is 65 %)

The product can be further purified with mixture of toluene, acetonitrile as per prior art methods.

### Example-4: Preparation of 1-[[[(1R)-1-[3-[(1E)-2-(7-chloro-2-quinolinyl) ethenyl] phenyl]-3-[2-(1-hydroxy-1-methylethyl) phenyl] propyl] thio] methyl] cyclopropane acetic acid dipropylamine salt (Montelukast DPA salt)

Similarly Montelukast dipropylamine salt is prepared by following the same procedure as in Example-III, by using the dipropyl amine instead of dicyclohexylamine followed by addition of n-Heptane in place of n-hexane affords the Montelukast dipropylamine, weight 82 g (yield 78 %)
Elemental analysis: C: 71.79%, H: 7.58%, N: 4.19%, S: 4.33% and calculated values for C₄₁H₅₁ClN₂O₃S C: 71.64%, H: 7.48%, N: 4.08%, S: 4.66%
IR Spectrum (KBr, cm-¹): 3210, 3031, 2973, 2930, 2865, 1627, 1607, 1593, 1494, 1409, 1374, 1340, 1282, 1181, 1154, 1138, 1128,1068, 1049, 1018, 963, 938, 860, 831, 757, 691.
¹H NMR (300 MHz, CDCl₃, ppm): 8.09-8.12 (d, 1H), 8.05- 8.06 (d, 1H), 7.65 - 7.73 (m, 3H), 7.08 - 7.48 (m, 10H), 3.99 (t, 1H), 3.14 - 3.24 (m, 1H), 2.83 - 2.93 (m, 1H), 2.36 - 2.71 (m, 6H), 2.05 - 2.30 (m, 4H), 1.56 -1.68 (m, 10H), 0.92 (t, 6H), 0.34 - 0.55 (m, 4H).
Mass Spectrum (M+): 586.2 (as free acid)

### Example-5: Preparation of 2-[2-[(3S)-[3-[(2E)-(7-chloroquinolin-2-yl) ethenyl] phenyl]-3-chloropropyl] phenyl-2-propanol (Chloro alcohol, VII, X=C1):

### Step-1:

Methyl 2-[(3S)-[3-[(2E)-(7-chloro quinolin-2-yl) ethenyl] phenyl]-3-chloropropyl] benzoate (100 g) is suspended in toluene (900ml) and raised the temperature to 108°C - 110°C, dehydrated by azeotropic distillation and cooled the solution to 20°C - 25°C.

### Step-2:

Cerium chloride (50g) is suspended in THF (1050 ml), raised the temperature of the suspension and distilled off initially 50 ml of THF and maintained the mass at reflux temperature (65°C) for 3 hrs under nitrogen atmosphere. Cooled the reaction mass to -5°C, add 3.0 Molar methyl magnesium chloride solution in THF (500 ml) at temperature -5°C - 0°C over 40 min and maintained for 2 hrs at that temperature. Added step-1 solution to this reaction mass slowly at 0°C - 5°C and maintained for 2 hrs at temperature of 0°C - 5°C. Transferred the reaction mass into a pre cooled mixture of 12% acetic acid (1400 ml): ethyl acetate (800 ml) at temperature below 20°C and mixed for 30 min at 18°C - 20°C. Allowed to settle, separated the organic layer, extracted the aqueous layer with ethyl acetate (800 ml), combined organic layer wash successively with 10% sodium carbonate solution (1600 ml), 5 % sodium chloride solution (2 x 800 ml) and dried the organic layer over anhydrous sodium sulphate (15g). Treated the dried organic layer with activated carbon, distilled off ethyl acetate from the clear solution at temperature below 45°C under pressure, added ethyl acetate (200 ml) and again distilled off under reduced pressure to get the solid. To the solid added ethyl acetate (100 ml), raised, maintained the temperature at 50°C - 55°C for about 30 min, cooled and maintained at 0°C - 5°C for 30 min. Filtered the product, washed with pre-cooled ethyl acetate (50 ml) and dried at 45°C - 50°C.

The dry weight of the chloro alcohol is 65g (yield is 65 %)
Elemental analysis: C: 73.16%, H: 5.68%, N: 3.14% and calculated values for C₂₉H₂₇Cl₂NO C: 73.10%, H: 5.67%, N: 2.94%
IR Spectrum (KBr, cm-¹): 3367, 3054, 2968, 2931, 1641, 1608, 1595, 1497, 1444, 1410, 1371, 1312, 1238, 1226, 1150, 1132, 1070, 963, 930, 880, 865, 837, 763, 754, 697, 671, 625, 592
¹H NMR (300 MHz, CDCl₃, ppm): 8.12 (d, 1H), 8.09 (d, 1H), 7.65-7.75 (m, 4H), 7.57 - 7.59 (m, 1H), 7.38 - 7.49 (m, 5H), 7.17 - 7.25 (m, 3H), 5.02 - 5.07 (dd, 1H), 4.15 (s, 1H), 3.00 - 3.35 (td, 2H), 2.39 - 2.54 (m, 2H), 1.69 (s, 3H), 1.67 (s, 3H).
Mass Spectrum (M+): 476.1

### Example-6: Preparation of 1-[[[(1R)-1-[3-[(1E)-2-(7-chloro-2-quinolinyl) ethenyl] phenyl]-3-[2-(1-hydroxy-1-methylethyl) phenyl] propyl] thio] methyl] cyclopropaneacetic acid dicyclohexylamine salt (Montelukast DCHA salt)

Sodium hydride (28 g, 0.70 moles) is suspended in DMF (200 ml) and cooled the suspension to -5°C under nitrogen, slowly added the solution of 1-(mercaptomethyl) cyclopropaneacetic acid (46 g, 0.315 mole) in DMF (100 ml) at -5°C to 0°C over 1 hr and maintained at -5°C to 0°C for 1 hr. Then slowly added the solution of 2-[2-[(3S)-[3-[(2E)-(7-chloroquinolin-2-yl) ethenyl] phenyl]-3-chloropropyl] phenyl -2-propanol (100 g, 0.21 mole) in DMF (300 ml) at -5°C to 0°C over 1hr and maintained the reaction mass at -5°C to 0°C for 12 hrs. Transferred the reaction mass into a mixture of water (1000 ml): ethyl acetate (1000 ml) and mixed for 30 min at temperature below 20°C. Adjusted the pH of the reaction mass to 7.0 by addition of 20% aqueous solution of tartaric acid at 20°C-25°C and mixed for 30 min. Reaction mixture is allowed for settling, separated the organic layer and extracted the aqueous layer with ethyl acetate (1000 ml). Combined the organic layer with ethyl acetate extraction, washed with 5% aqueous tartaric acid solution (400 ml) followed by water (2 x 1000 ml), dried over sodium sulphate, treated with activated carbon for 30 min at 25°C - 35°C, filtered the mass and distilled off ethyl acetate under reduced pressure at temperature below 40°C to get the residue. Dissolved the residue in ethyl acetate (600 ml) by heating to 45°C, cooled to 20°C and slowly added the dicyclohexyl amine (42 ml, 0.21 mole) over 45 min. at 20°C. Maintained at 20°C - 22°C for 1 hr, seeded with Montelukast DCHA salt (500 mg) and maintained at 20°C - 25°C for 24 hrs. Slowly added n-Hexane (1200 ml) over 60 min, maintained the reaction mass for 24 hrs at 20°C - 25°C. Filtered the solid, washed with n-Hexane (500 ml) and dried at 40°C - 45°C till constant weight.

Dry wt of the Montelukast DCHA salt is 65 g, (yield of 40.5%).

The product is identical with the product obtained as per the prior art methods.

### Example-7: Preparation of 1-[[[(1R)-1-[3-[(1E)-2-(7-chloro-2-quinolinyl) ethenyl] phenyl]-3-[2-(1-hydroxy-1-methylethyl) phenyl] propyl] thio]methyl]cyclopropaneacetic acid Alpha-methylbenzylamine salt (Montelukast MBA salt)

Montelukast alpha-methylbenzylamine salt can be prepared similarly by following the same procedure as in example-VI, using the alpha-methylbenzylamine instead of dicyclohexylamine affords the Montelukast alpha-methylbenzylamine salt, weight 70 g (yield 47.1 %)
Elemental analysis: C: 72.69%, H: 6.90%, N: 4.21%, S: 4.50% and calculated values for C₄₃H₄₇ClN₂O₃S C: 73.01%, H: 6.70%, N: 3.96%, S : 4.53%
IR Spectrum (KBr, cm-¹): 3400, 2976, 2927, 1607, 1594, 1578, 1541, 1497, 1410, 1394, 1336, 1311 1269, 1144, 1070, 1018, 965, 865, 840, 763, 699.
¹H NMR (300 MHz, CDCl₃, ppm): 8.13 - 8.15 (d, 1H), 8.06 - 8.07 (d, 1H), 7.63 - 7.81 (m, 4H), 7.12 - 7.53 (m, 14H) 4.12 - 4.16 (q, 1H), 4.04 (t, 1H), 3.16 - 3.23 (m, 1H), 2.91 - 2.99 (m, 1H), 2.18 - 2.73 (m, 6H), 1.65 (s, 3H), 1.63 (s, 3H), 1.42 - 1.44 (d, 3H), 0.46 - 0.59 (m, 4H).
Mass Spectrum (M+): 586.2 (as free acid)

### Example-8: Preparation of 1-[[[(1R)-1-[3-[(1E)-2-(7-chloro-2-quinolinyl) ethenyl] phenyl]-3-[2-(1-hydroxy-1-methylethyl) phenyl] propyl] thio] methyl] cyclopropaneacetic acid (Montelukast free acid)

Montelukast free acid is prepared from 2-[2-[(3S)-[3-[(2E)-(7-chloroquinolin-2-yl) ethenyl] phenyl]-3-chloropropyl]phenyl-2-propanol by following the same procedure as in example-VI, used ethanol instead of ethyl acetate for dissolution of residue, raised the temperature and maintained the temperature at reflux for 30 min, gradually cooled the mass to 28°C - 32°C, seeded with Montelukast free acid (500mg) and maintained for 24 hrs at 28°C - 32°C, cooled the mass to 20°C , maintained for 1hr, filtered the product and dried till constant weight.
Dry weight of the Montelukast free acid: 43 g (yield 70 %)
Elemental analysis: C: 71.2%, H: 6.22%, N: 2.41%, S: 5.40% and calculated values for C₃₅H₃₆ClNO₃S C: 71.70%, H: 6.19%, N: 2.39%, S: 5.47%
IR Spectrum (KBr, cm-¹): 3417, 2972, 2925, 1708, 1608, 1498, 1441, 1313, 1145, 1074, 963, 937, 863, 838, 762, 697.
¹H NMR (300 MHz, CDCl₃, ppm): 8.11(d, 1H), 8.07 (d, 1H), 7.73 (brs, 1H), 7.61 - 7.74 (m, 4H), 7.45 (m, 1H), 7,43 - 7.53 (dd, 1H), 7.33 - 7.43 (m, 3H), 7.10 - 7.20 (m, 3H), 5.02 - 5.07 (t, 1H), 3.0 - 3.35 (m, 2H), 2.65 (m, 2H), 2.40 - 2.54 (m, 2H), 2.39 (m, 2H), 1.63 (2s, 6H), 0.51 - 0.61 (m, 4H).
Mass Spectrum (M⁺): 586.2 [M⁺H]

### Example - 9: Preparation of Montelukast free acid from Montelukast DPA salt

Montelukast DPA salt (100 g, 0.146 mole) is suspended in a mixture of methylene chloride (1200 ml), water (700 ml) and mixed for 15 min. 6% acetic acid (193 ml) is added at temperature of 25°C - 35°C, mixed for 30 min, allowed for settling and separated the layers. Extracted the aqueous layer with methylene chloride (700 ml) and combined the organic layers. Washed the combined organic layer with water (700 ml), dried over sodium sulphate and distilled off methylene chloride completely to get residue. Added ethyl acetate (160 ml) and raised the temperature to reflux. Gradually cooled the reaction mass to 20°C - 25°C, seeded with Montelukast free acid (500 mg) and maintained at 20°C - 25°C for 12 hrs. Filtered the product, washed with chilled ethyl acetate (50 ml) and dried at 45°C - 50°C till constant weight.

Dry wt of Montelukast free acid is 60 g (70.3%)

### Example- 10: Preparation of Montelukast sodium from Montelukast free acid

Dissolved Montelukast free acid (100 g) in methanol (800 ml) by raised the temperature to 50°C, cooled the clear solution to 25°C - 30°C and added 0.486 molar solution of 1% aqueous ethanol solution (352 ml) over 30 min. Maintained the mass at 25°C - 30°C for 30 min. and treated the solution with activated carbon, Filtered off the carbon, distilled the solvents from filtrate at temperature below 40°C under reduced pressure to get residue. Added toluene (100 ml) and again distilled off under reduced pressure to remove traces of methanol, Ethanol. Dissolved the residue in toluene (1000 ml), raised the temperature and maintained at 45°C - 50°C. Cooled the solution to 30°C - 35°C, added carbon, mixed for 15 min and filtered off the carbon. Added n-Heptane (3000 ml) slowly to the clear filtrate over 1 hr at temperature 25°C - 30°C and maintained for 3 hrs. Filtered the product, washed with n-Heptane (50 ml) and dried at 80°C - 90°C under vacuum till constant weight.

Dry weight of Montelukast sodium is 90 g (87%)

The product is identical with the Montelukast sodium obtained in the prior art methods.

### Example-11: Preparation of Montelukast sodium from Montelukast DPA salt

Suspended Montelukast DPA salt (100 g, 0.146 mole) in a mixture of methylene chloride (2000 ml), water (1500 ml) and mixed for 15 min. Added 6% acetic acid (216 ml) at temperature of 25°C - 35°C, mixed for 30 min, allowed to settle and separated the layers. Extracted the aqueous layer with methylene chloride (1000 ml) and combined the organic layers. Washed the combined organic layer with water (1500 ml), dried over sodium sulphate and treated with carbon for 15 min. at 25°C - 30°C. Filtered off the carbon and added 0.486 molar sodium hydroxide solution in ethanol (275 ml) at 25°C - 30°C over 30 min. Maintained for 30 min at 25°C - 30°C and distilled methylene chloride at temperature below 40°C till to get residue under reduced pressure. Added toluene (200 ml) and distilled under vacuum at temperature below 40°C to get residue. Added toluene (800 ml) to the residue mixed for 15 min and treated with activated carbon at 25°C - 35°C for 20 min. Filtered off the carbon and washed the carbon bed with toluene (200 ml). Poured the clear filtrate slowly into n-Heptane (3000 ml) over 1 hr at 25°C - 35°C under nitrogen. Maintained at 25°C - 30°C for 2 hrs, filtered the product, washed with n-Heptane (100 ml) and dried at 90°C - 95°C under vacuum till constant weight.
Dry weight of Montelukast sodium is 60 g (yield: 67.8 %)

### Example-12: Preparation of Montelukast sodium from Montelukast MBA Salt

Suspended Montelukast MBA salt (100 g, 0.14 mole) in a mixture of methylene chloride (2000 ml) and water (1500 ml), mixed for 15 min. Added 6% acetic acid (210 ml, 1.48 mol equiv.) at temperature of 25°C - 35°C, mixed for 30 min, allowed to settle and separated the layers. Extracted the aqueous layer with methylene chloride (1000 ml) and combined the organic layers. Washed the combined organic layer with water (1500 ml), dried over sodium sulphate and treated with activated carbon for 15 min. at 25°C - 30°C. Filtered off the carbon and added 0.486 molar sodium hydroxide solution in ethanol (267 ml) at 25°C - 30°C over 30 min. Maintained for 30 min at 25°C - 30°C and distilled off methylene chloride at temperature below 40°C till to get residue finally under reduced pressure. Added toluene (200 ml) and distilled off under vacuum at temperature below 40°C to get residue. Added toluene (800 ml) to the residue mixed for 15 min. and treated with carbon at 25°C - 35°C for 20 min. Filtered off the carbon and washed the carbon bed with toluene (200 ml). Poured the clear filtrate slowly into n-Heptane (3000 ml) over 1 hr at 25°C - 35°C under nitrogen. Maintained at 25°C - 30°C for 2 hrs, filtered the product, washed with n-Heptane (100 ml) and dried at 90°C - 95°C under vacuum till constant weight.
Dry weight ofMontelukast sodium is 65 g (yield: 75.6 %)

## Claims

1. A process for the preparation of Montelukast free acid and its alkali salts without the formation of unstable or limited stable intermediates comprising
- reacting methyl 2-[(3S)-[3-[(2E)-(7-chloroquinolin-2-yl) ethenyl] phenyl]-3-halopropyl] benzoate with 1-(mercaptomethyl) cyclopropane acetic acid in the presence of alkali hydrides or alkoxides to yield 2-[1-[1(R)-[3-[2-(7-chloroquinolin-2-yl) ethenyl] phenyl]-3-[2-(methoxycarbonyl) phenyl] propyl sulfanylmethyl] cyclopropane acetic acid, which on reaction with a Grignard reagent gives Montelukast free acid or, optionally,
- reacting methyl 2-[(3S)-[3-[(2E)-(7-chloroquinolin-2-yl) ethenyl] phenyl]-3-halopropyl] benzoate with a Grignard reagent to yield 2-[2-[(3S)-[3-[(2E)-(7-chloroquinolin-2-yl) ethenyl] phenyl]-3-halopropyl] phenyl-2-propanol, which on condensation with 1-(mercaptomethyl) cyclopropane acetic acid in the presence of alkali hydrides or alkoxides gives Montelukast free acid, and
- isolation of the Montelukast as Montelukast free acid or optionally as Montelukast organic base salts,

2. A process as claimed in claim 1, wherein the term halo in methyl-2-[(3S)-[3-[(2E)-(7-chloroquinolin-2-yl) ethenyl] phenyl]-3-halopropyl] benzoate or 2-[2-[(3S)-[3-[(2E)-(7-chloroquinolin-2-yl) ethenyl] phenyl]-3-halopropyl] phenyl-2-propanol represents chloro, bromo, or iodo.

3. A process as claimed in claim 1, wherein the alkali hydride is sodium hydride or the alkali alkoxide is potassium tert-butoxide.

4. A process as claimed in claim 1, wherein the reaction of methyl 2-[(3S)-[3-[(2E)-(7-chloroquinolin-2-yl) ethenyl] phenyl]-3-halopropyl] benzoate (halo ester) with 1-(mercapto methyl) cyclopropane acetic acid is carried out in the presence of a solvent.

5. A process as claimed in claim 1, wherein the reaction of 2-[2-[(3S)-[3-[(2E)-(7-chloroquinolin-2-yl) ethenyl] phenyl]-3-halopropyl] phenyl-2-propanol with 1-(mercaptomethyl) cyclopropane acetic acid is carried out in an organic solvent.

6. A process as claimed in claim 4 or 5, wherein the organic solvent is dimethyl formamide or tetrahydrofuran.

7. A process as claimed in claim 1, wherein 2-[1-[1(R)-[3-[2-(7-chloroquinolin-2-yl) ethenyl] phenyl]-3-[2-(methoxycarbonyl) phenyl] propyl sulfanyl methyl] cyclopropane] acetic acid is isolated as its organic base salt.

8. A process as claimed in claim 7, wherein the 2-[1-[1(R)-[3-[2-(7-chloroquinolin-2-yl) ethenyl] phenyl]-3-[2-(methoxycarbonyl) phenyl] propyl sulfanyl methyl] cyclopropane] acetic acid organic base salt is the dicyclohexyl amine salt.

9. A process as claimed in claim 1, wherein the Grignard reagent is selected from methyl magnesium chloride and methyl magnesium bromide.

10. A process as claimed in claim 1, wherein the Montelukast organic base salts are selected from Montelukast dipropylamine salt, Montelukast alpha methylbenzylamine salt, Montelukast dibenzylamine salt, Montelukast dicyclohexylamine salt and Montelukast di-isopropylamine salt.

11. A process for the preparation of Montelukast organic base salts from Montelukast free acid comprising
- dissolving Montelukast free acid obtained by the process of claim 1 in ethyl acetate,
- cooling the reaction mass to a temperature of 20°C to 35°C,
- adding an organic base,
- maintaining the reaction mass at this temperature for 10 hrs to 36 hrs,
- adding the second solvent,
- mixing the reaction mass for 2 hrs to 18 hrs,
- isolating and drying of Montelukast organic base salts.

12. A process as claimed in claim 11, wherein the organic base is dicyclohexylamine, dipropylamine, di-isopropylamine, dibenzylamine or alpha methyl benzylamine.

13. A process as claimed in claim 11, wherein the second solvent is a C-5 to C-7 hydrocarbon, acetonitrile, or C-4 to C-8 ethers.

14. A process for the preparation of Montelukast sodium from Montelukast free acid comprising
- dissolving Montelukast free acid obtained by the process of claim 1 in methanol,
- cooling the reaction mass to a temperature of 20°C to 35°C,
- adding a sodium hydroxide solution in ethanol,
- maintaining the reaction mass at this temperature for 30 min to 2 hrs,
- removing the solvents at a temperature below 40°C,
- adding toluene to the residue,
- dissolving the residue in toluene by raising the temperature to 40°C to 60°C,
- cooling the reaction mass to a temperature of 20°C to 35°C,
- pouring the toluene solution into n-heptane at a temperature of 20°C to 35°C,
- mixing the reaction mass for 2 hrs to 18 hrs,
- isolating and drying of Montelukast sodium.

15. A process for the preparation of Montelukast sodium from Montelukast organic base salts comprising
- suspending the Montelukast organic base salt obtained by the process of claim 11 in a mixture of water and methylene chloride,
- adding an acetic acid solution,
- separating the layers,
- washing the organic layer with water,
- adding a sodium hydroxide solution in ethanol,
- removing methylene chloride,
- adding toluene,
- transferring the toluene solution to n-heptane,
- isolating and drying of Montelukast sodium.

16. A process as claimed in claim 15, wherein the Montelukast organic base salt is Montelukast dipropylamine salt, Montelukast dibenzyamine salt, Montelukast alpha methyl benzylamine salt, Montelukast dicyclohexylamine salt or Montelukast diisopropylamine salt.

17. A process for the preparation of Montelukast free acid from Montelukast organic salts comprising
- suspending the Montelukast organic base salt obtained by the process of claim 11 in a mixture of water and methylene chloride,
- adding an acetic acid solution,
- separating the layers,
- washing the organic layer with water,
- removing methylene chloride,
- dissolving the residue in ethyl acetate,
- cooling the reaction mass,
- isolating and drying the Montelukast free acid.

18. A process as claimed in claim 17, wherein the Montelukast organic base salt is Montelukast dipropylamine salt, Montelukast dibenzylamine salt, Montelukast alpha methylbenzylamine salt, Montelukast di-isopropylamine salt or Montelukast dicyclohexylamine salt.

19. A compound 2-[2-[(3S)-[3-[(2E)-(7-chloroquinolin-2-yl) ethenyl] phenyl]-3-halopropyl] phenyl-2-propanol.

20. A compound as claimed in claim 16, wherein the term halo represents chloro, bromo or iodo.

## Patentansprüche

1. Verfahren zur Herstellung von Montelukast in Form seiner freien Säure oder in Form ihrer Alkalimetallsalze ohne Bildung von instabilen Intermediaten oder solchen mit begrenzter Stabilität, umfassend
- Reaktion von Methyl-2-[(3S)-[3-[(2E)-(7-chlorchinolin-2-yl)-ethenyl]-phenyl]-3-halopropyl]-benzoat mit 1-Mercaptomethylcyclopropylessigsäure in Gegenwart von Alkalimetallhydriden oder -alkoxiden zum Erhalt von 2-[1-[1(R)-[3-[2-(7-Chlorchinolin-2-yl)-ethenyl]-phenyl]-3-[2-methoxycarbonylphenyl]-propylsulfanylmethyl]-cyclopropylessigsäure, die nach Reaktion mit einem Grignard-Reagenz Montelukast in Form seiner freien Säure liefert oder optional
- Reaktion von Methyl-2-[(3S)-[3-[(2E)-(7-chlorchinolin-2-yl)-ethenyl]-phenyl]-3-halopropyl]-benzoat mit einem Grignard-Reagenz, um 2-[2-[(3S)-[3-[(2E)-(7-Chlorchinolin-2-yl)-ethenyl]-phenyl]-3-halopropyl]-phenyl-2-propanol zu erhalten, das unter Kondensation mit 1-Mercaptomethylcyclopropylessigsäure in Gegenwart von Alkalimetallhydriden oder -alkoxiden Montelukast in Form seiner freien Säure liefert, und
- Isolierung von Montelukast in Form seiner freien Säure oder, optional, in Form eines Salzes mit einer organischen Base.

2. Verfahren nach Anspruch 1, wobei "Halo" in Methyl-2-[(3S)-[3-[(2E)-(7-chlorchinolin-2-yl)-ethenyl]-phenyl]-3-halopropyl]-benzoat oder in 2-[2-[(3S)-[3-[(2E)-(7-Chlorchinolin-2-yl)-ethenyl]-phenyl]-3-halopropyl]-phenyl-2-propanol für Chlor, Brom oder Iod steht.

3. Verfahren nach Anspruch 1, wobei das Alkalimetallhydrid Natriumhydrid oder das Alkalimetallalkoxid Kalium-tert-butoxid ist.

4. Verfahren nach Anspruch 1, wobei die Reaktion von Methyl-2-[(3S)-[3-[(2E)-(7-chlorchinolin-2-yl)-ethenyl]-phenyl]-3-halopropyl]-benzoat (Haloester) mit 1-Mercaptomethylcyclopropylsäure in der Gegenwart eines Lösungsmittels durchgeführt wird.

5. Verfahren nach Anspruch 1, wobei die Reaktion von 2-[2-[(3S)-[3-[(2E)-(7-Chlorchinolin-2-yl)-ethenyl]-phenyl]-3-halopropyl]-phenyl-2-propanol mit 1-Mercaptomethylcyclopropansäure in einem organischen Lösungsmittel durchgeführt wird.

6. Verfahren nach Anspruch 4 oder 5, wobei das organische Lösungsmittel Dimethylformamid oder Tetrahydrofuran ist.

7. Verfahren nach Anspruch 1, wobei die 2-[1-[1(R)-[3-[2-(7-Chlorchinolin-2-yl)-ethenyl]-phenyl]-3-[2-methoxycarbonylphenyl]-propylsulfanylmethyl]-cyclopropylessigsäure in Form eines Salzes mit einer organischen Base isoliert wird.

8. Verfahren nach Anspruch 7, wobei das Salz der 2-[1-[1(R)-[3-[2-(7-Chlorchinolin-2-yl)-ethenyl]-phenyl]-3-[2-methoxycarbonylphenyl]-propylsulfanylmethyl]-cyclopropylessigsäure das Dicyclohexylaminsalz ist.

9. Verfahren nach Anspruch 1, wobei das Grignard-Reagenz ausgewählt ist aus der Gruppe Methylmagnesiumchlorid und Methylmagnesiumbromid.

10. Verfahren nach Anspruch 1, wobei das Salz des Montelukasts mit einer organischen Base ausgewählt ist aus dem Diisopropylamin-, alpha-Methylbenzylamin-, Dibenzylamin-, Dicyclohexylamin- und dem Diisopropylaminsalz des Montelukasts ausgewählt ist.

11. Verfahren zur Herstellung eines Salzes des Montelukasts mit einer organischen Base aus Montelukast in Form seiner freien Säure umfassend
- Lösen von Montelukast in Form seiner freien Säure erhalten nach dem Verfahren gemäß Anspruch 1 in Ethylacetat,
- Kühlen des Reaktionsgemischs auf eine Temperatur von 20 °C bis 35 °C,
- Zugabe einer organischen Base,
- Halten des Reaktionsgemischs bei dieser Temperatur für 10 bis 30 Stunden,
- Zugabe eines zweiten Lösungsmittels,
- Mischen des Reaktionsgemischs für 2 bis 18 Stunden,
- Isolierung und Trocknung des Salzes des Montelukasts mit der organischen Base.

12. Verfahren nach Anspruch 11, wobei die organische Base Dicyclohexylamin, Dipropylamin, Diisopropylamin, Dibenzylamin oder alpha-Methylbenzylamin ist.

13. Verfahren nach Anspruch 11, wobei das zweite Lösungsmittel ein C₅-C₇-Kohlenwasserstoff-, Acetonitril- oder ein C₄-C₈-Ether ist.

14. Verfahren zur Herstellung des Natriumsalzes des Montelukasts aus Montelukast in Form seiner freien Säure umfassend
- Lösen von Montelukast in Form seiner freien Säure erhalten nach Anspruch 1 in Methanol,
- Kühlen des Reaktionsgemischs auf eine Temperatur von 20 °C bis 35 °C,
- Zugabe einer Natriumhydroxidlösung in Ethanol,
- Halten des Reaktionsgemischs bei dieser Temperatur für 30 Minuten bis 2 Stunden,
- Entfernen der Lösungsmittel bei einer Temperatur unterhalb von 40 °C,
- Zugabe von Toluol zum Rückstand,
- Lösen des Rückstands in Toluol unter Erhöhung der Temperatur auf 40 °C bis 60 °C,
- Kühlen des Reaktionsgemischs auf eine Temperatur von 20 °C bis 35 °C,
- Aufnahme der Toluol-Lösung in n-Heptan bei einer Temperatur von 20 °C bis 35 °C,
- Mischen des Reaktionsgemischs für 2 bis 18 Stunden,
- Isolierung und Trocknung des Natriumsalzes des Montelukasts.

15. Verfahren zur Herstellung von Montelukast-Natrium aus einem Salz des Montelukasts mit einer organischen Base umfassend
- Suspendieren des Salzes des Montelukasts mit einer organischen base erhalten gemäß dem Verfahren aus Anspruch 11 in einer Mischung aus Wasser und Methylenchlorid,
- Zugabe einer Essigsäurelösung,
- Trennung der Phasen,
- Waschen der organischen Phase mit Wasser,
- Zugabe einer Natriumhydroxidlösung in Ethanol,
- Entfernen des Methylenchlorids,
- Zugabe von Toluol,
- Aufnahme der Toluol-Lösung in n-Heptan,
- Isolierung und Trocknung von Montelukast-Natrium.

16. Verfahren nach Anspruch 15, wobei das Salz des Montelukasts mit einer organischen Base das Dipropylamin-, Dibenzylamin-, alpha-Methylbenzylamin-, Dicyclohexylamin- oder das Diisopropylamin-Salz des Montelukasts ist.

17. Verfahren zur Herstellung von Montelukast in Form seiner freien Säure ausgehend von einem Salz des Montelukasts mit einer organischen Base umfassend
- Suspendieren des Salzes des Montelukasts mit einer organischen Base erhalten gemäß dem Verfahren nach Anspruch 11 in einer Mischung aus Wasser und Methylenchlorid,
- Zugabe einer Essigsäurelösung,
- Trennen der Phasen,
- Waschen der organischen Phase mit Wasser,
- Entfernen des Methylenchlorids,
- Lösen des Rückstands in Ethylacetat,
- Kühlen des Reaktionsgemischs,
- Isolieren und Trocknen von Montelukast in Form seiner freien Säure.

18. Verfahren nach Anspruch 17, wobei das Salz des Montelukasts mit einer organischen Base das Dipropylamin-, Dibenzylamin-, alpha-Methylbenzylamin-, Diisopropylamin- oder das Dicyclohexylamin-Salz des Montelukasts ist.

19. Verbindung der Formel 2-[2-[(3S)-[3-[(2E)-(7-Chlorchinolin-2-yl)-ethenyl]-phenyl]-3-halopropyl)-phenyl-2-propanol.

20. Verbindung nach Anspruch 19, wobei "Halo" für Chlor, Brom oder Iod steht.

## Revendications

1. Procédé de préparation d'acide libre de montelukast et de ses sels alcalins sans la formation de produits intermédiaires instables ou à stabilité limitée, comprenant les étapes consistant à :
- faire réagir du méthyl-2-[(3S)-[3-[(2E)-(7-chloroquinolin-2-yl)-éthényl]phényl]-3-halopropyl]benzoate avec de l'acide acétique de 1-(mercaptométhyl)cyclopropane en présence d'hydrures ou d'alcoxydes alcalins pour produire de l'acide acétique de 2-[1-[1(R)-[3-[2-(7-chloroquinolin-2-yl)éthényl]phényl]-3-[2-(méthox ycarbonyl)phényl]propylsulfanylméthyl]cyclopropane qui, en réaction avec un réactif de Grignard, donne de l'acide libre de montelukast ou, éventuellement,
- faire réagir du méthyl-2[(3S)-[3-(2E)-(7-chloroquinolin-2-yl)éthényl]-phényl]-3-halopropyl]benzoate avec un réactif de Grignard pour produire du 2-[2-(3S)-[3-[(2E)-(7-chloroquinolin-2-yl)éthényl]-phényl]-3-halopropyl]phényl-2-propanol qui, par condensation avec de l'acide acétique de 1-(mercaptométhyl)cyclopropane en présence d'hydrures ou d'alcoxydes alcalins, donne de l'acide libre de montelukast, et
- isoler le montelukast sous la forme d'acide libre de montelukast ou éventuellement sous la forme de sels basiques organiques de montelukast.

2. Procédé selon la revendication 1, dans lequel le terme halogéno dans le méthyl-2-[(3S)-[3-[(2E)-(7-chloroquinolin-2-yl)éthényl]phényl]-3-halopropyl] benzoate ou le 2-[2-[(3S)-[3-[(2E)-(7-chloroquinolin-2-yl)-éthényl]phényl]-3-halopropyl]phényl-2-propanol représente un groupe chloro, bromo ou iodo.

3. Procédé selon la revendication 1, dans lequel l'hydrure alcalin est un hydrure de sodium ou l'alcoxyle alcalin est du tert-butoxyde de potassium.

4. Procédé selon la revendication 1, dans lequel la réaction de méthyl-2[(3S)-[3-[(2E)-(7-chloroquinolin-2-yl)éthényl]phényl]-3-halopropyl]b enzoate (halogénoester) avec de l'acide acétique de 1-(mercaptométhyl)cyclopropane est mise en oeuvre en présence d'un solvant.

5. Procédé selon la revendication 1, dans lequel la réaction de 2-[2-[(3S)-[3[(2E)-(7-chloroquinolin-2-yl)éthényl]phényl]-3-halopropyl]phén yl-2-propanol avec de l'acide acétique de 1-(mercaptométhyl)cyclopropane est mise en oeuvre dans un solvant organique.

6. Procédé selon la revendication 4 ou 5, dans lequel le solvant organique est du diméthylformamide ou du tétrahydrofuranne.

7. Procédé selon la revendication 1, dans lequel de l'acide acétique de 2-[1-[1(R)-[3-[2-(7-chloroquinolin-2-yl)éthényl]phényl]-3-[2-(méthoxycarbon yl)phényl]propylsulfanylméthyl]cyclopropane] est isolé sous la forme de son sel basique organique.

8. Procédé selon la revendication 7, dans lequel le sel basique organique d'acide acétique de 2-[1-[1(R)-[3-[2-(7-chloroquinolin-2-yl)éthényl]-phényl]-3[2-(méthoxycarbonyl)phényl]propylsulfanylméthyl]cyclopropane] est le sel de dicyclohexylamine.

9. Procédé selon la revendication 1, dans lequel le réactif de Grignard est sélectionné parmi du chlorure de méthylmagnésium et du bromure de méthylmagnésium.

10. Procédé selon la revendication 1, dans lequel les sels basiques organiques de montelukast sont sélectionnés parmi un sel de dipropylamine de montelukast, un sel d'alphaméthylbenzylamine de montelukast, un sel de dibenzylamine de montelukast, un sel de dicyclohexylamine de montelukast et un sel de diisopropylamine de montelukast.

11. Procédé de préparation de sels basiques organiques de montelukast à partir d'acide libre de montelukast comprenant les étapes consistant à :
- dissoudre de l'acide libre de montelukast obtenu par le procédé selon la revendication 1 dans de l'acétate d'éthyle,
- refroidir la masse réactionnelle jusqu'à une température de 20°C à 35°C,
- ajouter une base organique,
- maintenir la masse réactionnelle à cette température pendant 10 heures à 36 heures,
- ajouter le second solvant,
- mélanger la masse réactionnelle pendant 2 heures à 18 heures,
- isoler et sécher les sels basiques organiques de montelukast.

12. Procédé selon la revendication 11, dans lequel la base organique est de la dicyclohexylamine, de la dipropylamine, de la diisopropylamine, de la dibenzylamine ou de l'alphaméthylbenzylamine.

13. Procédé selon la revendication 11, dans lequel le second solvant est un hydrocarbure en C₅ à C₇, de l'acétonitrile, ou des éthers en C₄ à C₈.

14. Procédé de préparation de sodium de montelukast à partir d'acide libre de montelukast comprenant les étapes consistant à :
- dissoudre de l'acide libre de montelukast obtenu par le procédé selon la revendication 1 dans du méthanol,
- refroidir la masse réactionnelle jusqu'à une température de 20°C à 35°C,
- ajouter une solution d'hydroxyde de sodium dans de l'éthanol,
- maintenir la masse réactionnelle à cette température pendant 30 minutes à 2 heures,
- retirer les solvants à une température inférieure à 40°C,
- ajouter du toluène au résidu,
- dissoudre le résidu dans du toluène en augmentant la température jusqu'à 40°C à 60°C,
- refroidir la masse réactionnelle jusqu'à une température de 20°C à 35°C,
- verser la solution de toluène dans du n-heptane à une température de 20°C à 35°C,
- mélanger la masse réactionnelle pendant 2 heures à 18 heures,
- isoler et sécher le sodium de montelukast.

15. Procédé de préparation de sodium de montelukast à partir de sels basiques organiques de montelukast comprenant les étapes consistant à :
- mettre en suspension le sel basique organique de montelukast obtenu par le procédé selon la revendication 11 dans un mélange d'eau et de chlorure de méthylène,
- ajouter une solution d'acide acétique,
- séparer les couches,
- laver la couche organique avec de l'eau,
- ajouter une solution d'hydroxyde de sodium dans de l'éthanol,
- retirer le chlorure de méthylène,
- ajouter du toluène,
- transférer la solution de toluène dans du n-heptane,
- isoler et sécher le sodium de montelukast.

16. Procédé selon la revendication 15, dans lequel le sel basique organique de montelukast est un sel de dipropylamine de montelukast, un sel de dibenzylamine de montelukast, un sel d'alphaméthylbenzylamine de montelukast, un sel de dicyclohexylamine de montelukast ou un sel de diisopropylamine de montelukast.

17. Procédé de préparation d'acide libre de montelukast à partir de sels organiques de montelukast comprenant les étapes consistant à :
- mettre en suspension le sel basique organique de montelukast obtenu par le procédé selon la revendication 11 dans un mélange d'eau et de chlorure de méthylène,
- ajouter une solution d'acide acétique,
- séparer les couches,
- laver la couche organique avec de l'eau,
- retirer le chlorure de méthylène,
- dissoudre le résidu dans de l'acétate d'éthyle,
- refroidir la masse réactionnelle,
- isoler et sécher l'acide libre de montelukast.

18. Procédé selon la revendication 17, dans lequel l'acide basique organique de montelukast est un sel de dipropylamine de montelukast, un sel de dibenzylamine de montelukast, un sel d'alphaméthylbenzylamine de montelukast, un sel de diisopropylamine de montelukast ou un sel de dicyclohexylamine de montelukast.

19. Composé de 2-[2-[(3S)-[3-[(2E)-(7-chloroquinolin-2-yl)éthényl]-phényl]-3-halopropyl]phény 1-2-propanol.

20. Composé selon la revendication 16, dans lequel le terme halogéno représente un groupe chloro, bromo ou iodo.
